# EUROPEAN PATENT SPECIFICATION

(11) **EP 1 077 922 B1**
(45) Date of publication and mention of the grant of the patent: **18.02.2004**
(21) Application number: 99919567.0
(22) Date of filing: 13.05.1999
(51) Int. Cl.: C07C 211/14, C08L 95/00

(54) **POLYAMINE**
POLYAMINE
POLYAMINE

(30) Priority: 14.05.1998 JP 13212498
(43) Date of publication of application: 28.02.2001
(73) Proprietor: Kao Corporation, Tokyo 103-0025 (JP)
(72) Inventor: ISOBE, Kazuo, Wakayama-shi, Wakayama 640-8580 (JP); TAMAKI, Ryoichi, Wakayama-shi, Wakayama 640-8580 (JP); TOMIOKA, Keiichiro, Wakayama-shi, Wakayama 640-8580 (JP); YOSHIDA, Wataru, Wakayama-shi, Wakayama 640-8580 (JP); FUKUSHIMA, Tetsuaki, Wakayama-shi, Wakayama 640-8580 (JP); NISHIMOTO, Uichiro, Wakayama-shi, Wakayama 640-8580 (JP)
(74) Representative: HOFFMANN - EITLE
(86) International application number: PCT/JP1999/002486
(87) International publication number: WO 1999/058491

(56) References cited:
- EP-A- 0 125 852
- EP-A- 0 438 964
- FR-A- 2 211 449
- US-A- 3 251 664
- US-A- 3 781 363

## Description

### Technical Field

The present invention relates to a salt of a polyamine which is used in an asphalt emulsion, a catalyst for urethane, a chelating agent, a material for surfactants, a mining flotation agent, a material for fiber softeners and the like. The present invention also relates to an asphalt emulsion composition which is obtained by using the above-mentioned salt of a polyamine.

### Background Art

Heretofore, aliphatic amines, which have a straight-chain alkyl group having 12 to 22 carbon atoms, have been used in an emulsifier or the like for the production of an asphalt emulsion. However, since these amines are solids or pastes at normal temperature, the handling of these amines was not easy. Despite efforts, which have been made traditionally in order to liquefy these compounds, the following problems still remain.

For example, a significant sacrifice of surface activity is associated with the amines obtained by a process described in US-A 2930701 comprising oxyalkylating an alkylamine or an alkylpropylenediamine, or by a process described in US-A 4561900 comprising methylating a secondary nitrogen. That is, when these amines are used, the adding amount thereof needs to be larger than that of the solid amine as a material thereof, or alternatively, when these amines are used for the production of an emulsion, a larger amount of mechanical energy is required.

Meanwhile, when a road is paved with an asphalt emulsion, in order to open the road after paving operation thereof quickly to traffic, a method such as slurry seal or micro-surfacing is adopted. In this method, an asphalt emulsion, aggregates and water are loaded in a vehicle by a special mechanism which prevents their mutual contact. The asphalt emulsion, aggregates and water are mixed by a mixer while the vehicle moves and the mixture is spread on a road. In this method, when the asphalt emulsion, aggregates and water be mixed, it is desired that the vehicle runs and puts (or moves) easily on the mixture. Namely, the asphalt emulsion, aggregates and water should be mixed well and the mixture needfully has a sufficient fluidity (being as "miscibility of aggregates"). Further when the mixture is spread on a road, the demulsification desirably takes place as soon as possible so that the mixture sets (being as "quick hardenability"). A mixture, which sets within one hour after the spreading thereof so that the pavement is open to traffic, is described as having a quick setting property. Since the setting time significantly varies depending on the types of aggregates and temperatures, it is desired that the setting rate be controllable so that the mixture can be used under various conditions.

In order to meet the above-mentioned points and required performances, a variety of emulsifiers for asphalt and cationic asphalt emulsion compositions have been proposed.

For example, CA-A 953452 discloses a cationic asphalt emulsion in which a quaternary ammonium salt is used as an emulsifier, and US-A 5242492 describes a reaction product made from a fatty acid having 20 or more carbon atoms with a polyamine. However, none of these techniques satisfy the above-mentioned requirements.

EP 0 438 964 A1 discloses the preparation of specific polyamines comprising the cyanoethylation of a primary amine using acrylonitrile and hydrogenating the cyanoethylation product.

Accordingly, one objective of the present invention is to provide an amine salt which has a surface activity not inferior to that of an amine based on a solid tallow and an excellent workability and which is suited for use in the emulsification of asphalt or the like. Another objective of the present invention is to provide an asphalt emulsion composition which is obtained by using the amine salt and which has a quick setting property.

### Disclosure of Invention

The present inventors found that a specific polyamine is in a liquid state at normal temperature and the ability of the specific polyamine salt to emulsify asphalt is not reduced unlike the case of conventional liquid amines. In addition, they found that an asphalt emulsion composition containing a water-soluble salt of the specific polyamine has an excellent quick setting property; that is to say, the asphalt emulsion composition is excellent in the miscibility of aggregates; the setting time after paving operation can be controlled by the amounts added of fillers, such as cement, slaked lime and the like, and that of water; and the use of this emulsion composition makes it possible to open the road to traffic when one hour elapses after the paving operation under a wide range of conditions.

Accordingly, the present invention provides a salt of a polyamine represented by the formula (1) (hereinafter referred to as polyamine (1)), a process for producing the polyamine salt, use of the polyamine salt in an asphalt emulsion and an asphalt emulsion composition containing a salt of the amine preferably being water-soluble; 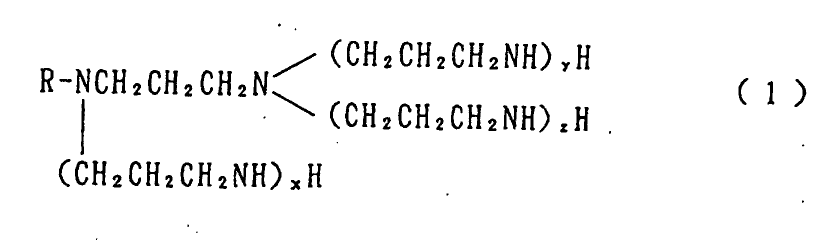 wherein R is a straight or branched hydrocarbon group having 8 to 22 carbon atoms; x is a number of 1 to 5; and each of y and z is a number of 0 to 5 with the proviso that both of y and z are not 0 at the same time.

A preferred salt of a polyamine for use in an asphalt emulsion is that prepared from an amine obtainable by carrying out the cyanoethylation of a compound represented by the formula (2) by reacting 0.2 to 3 moles of acrylonitrile with 1 mole of the compound or carrying out the cyanoethylation of a compound represented by the formula (3) by reacting 1.4 to (m+2) moles of acrylonitrile with 1 mole of the compound; and hydrogenating the cyanoethylation product.

The invention provides an asphalt emulsion composition containing a salt of the polyamine as above described and then an asphalt emulsion comprising asphalt, water and a salt of the polyamine.

The invention provides a method of emulsifying asphalt with a salt of the polyamine. Asphalt may be emulsified with the polyamine and an acid, preferably the polyamine being used in an equivalent or more to the acid.

The invention provides use of a salt of the polyamine as an emulsifier for asphalt.

### Modes for Carrying Out the Invention

In the salt of the polyamine (1) of the present invention, preferably R has 10 to 20 carbon atoms from the standpoint of emulsifiability and preferably R has 8 to 18 carbon atoms from the standpoint of being a liquid at normal temperature. From these standpoints, most preferably R has 10 to 18 carbon atoms. Besides, the hydrocarbon group may be made up of a mixture of hydrocarbon groups. X is preferably 1 to 2, and most preferably 1. The sum of y and z is preferably 1 to 4, and most preferably 1 to 3.

Examples of the polyamine (1) include the following compounds. Among them, from the standpoint of emulsifiability, preferable examples are (b), (c), (d), (h) and (i), and most preferable examples are (b), (c) and (d).

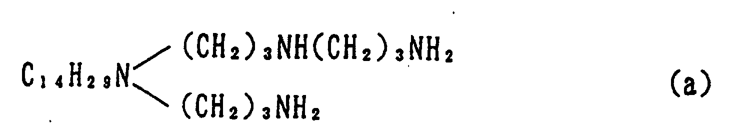

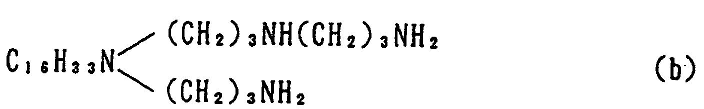

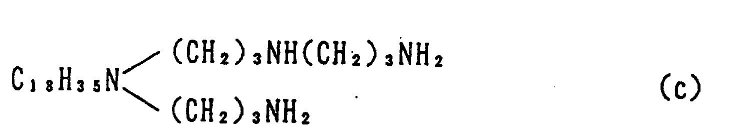

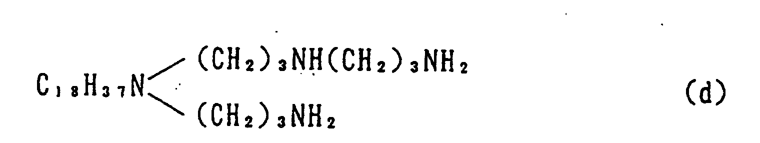

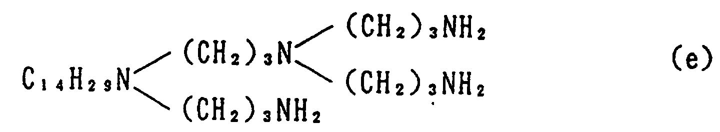

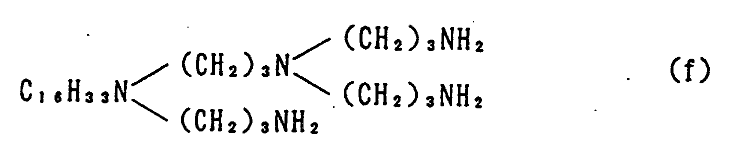

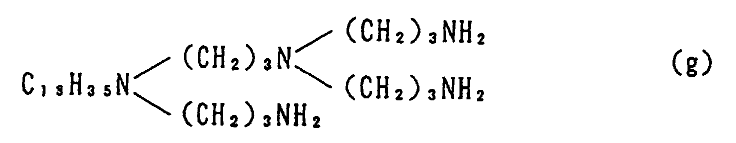

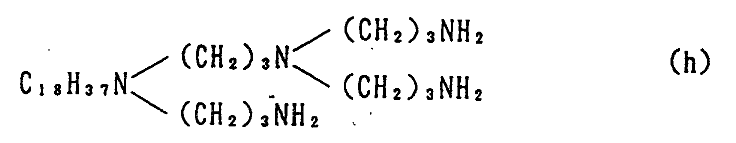

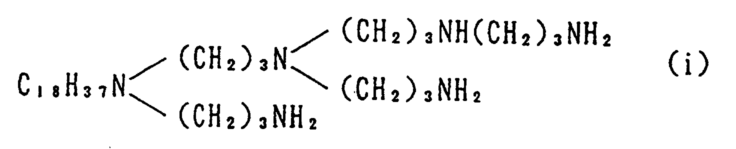

The polyamine (1) is obtained by carrying out the cyanoethylation of a compound represented by the formula (2) (hereinafter referred to as compound (2)) or a compound represented by the formula (3) (hereinafter referred to as compound (3)) by reacting acrylonitrile therewith and thereafter hydrogenating the cyanoethylation product;

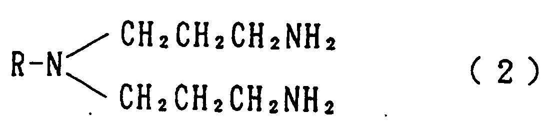

R-(NHCH₂CH₂CH₂)ₘ-NH₂ (3)

wherein R is as defined above; and m is a number of 1 to 3.

Specific examples of the compound (2) include N-myristyl-N-aminopropylpropylenediamine, N-stearyl-N-aminopropylpropylenediamine, N-tallow alkyl-N-aminopropylpropylenediamine and the like.

In the compound (3), m is 1 to 3 and is preferably 1 to 2 from the standpoint of emulsifiability. Besides, the group having m may be made up of a mixture of groups. Specific examples of the compound (3) include myristylpropylenediamine, stearylpropylenediamine, tallow alkylpropylenediamine, palm kernel oil alkylpropylenediamine, tallow alkyldipropylenetriamine, tallow alkyltripropylenetetramine and the like.

When the compound (2) is reacted with acrylonitrile, it is preferable that 0.2 to 3 moles of acrylonitrile be reacted per mole of the compound (2). Meanwhile, when the compound (3) is reacted with acrylonitrile, it is preferable that 1.4 to (m+3) moles of acrylonitrile be reacted per mole of the compound (3).

In the hydrogenating reaction of cyanoethylation product, the reaction temperature is preferable to be 100 to 160 °C from the standpoint of preventing by-product as small as possible.

Although the salt of the polyamine (1) is suited for use in an asphalt emulsion, a catalyst for urethane, a chelating agent, a material for surfactants, a mining flotation agent, a material for fiber softeners and the like, most preferably the salt of the polyamine (1) is used for an asphalt emulsion. When the salt of the amine (1) is used for an asphalt emulsion, the amine is preferably a reaction product obtained by a process comprising carrying out the cyanoethylation of a compound (2) by reacting 0.2 to 3 moles, more preferably 0.5 to 1.5 moles, of acrylonitrile with 1 mole thereof and hydrogenating the cyanoethylation product or alternatively by a process comprising carrying out the cyanoethylation of a compound (3) by reacting 1.4 to (m+2) moles, more preferably 1.8 to (m+2) moles, further preferably 2.1 to (m+2) moles from the stand point of liquefiability, of acrylonitrile with 1 mole thereof and hydrogenating the cyanoethylation product. When an alkylpropylenediamine, whose m is 1 in the formula (3), such as myristylpropylenediamine, stearylpropylenediamine., tallow alkylpropylenediamine or the like is used, the reaction molar number of the acrylonitrile for cyanoethylation is preferably in the range of from 1.4 to 3.0 from the standpoint of liquefiability of the amine to be obtained. Likewise, when a compound (3), whose m is 2, is used, the molar number of the acrylonitrile is preferably in the range of from 1.4 to 4.0. And, when a compound (3), whose m is 3, is used, the reaction molar number of the acrylonitrile is preferably in the range of from 1.4 to 5.0.

From the standpoint of being a liquid at 20°C and presenting an excellent workability, the solidification temperature of the polyamine (1) is preferably 20°C or below. The solidification temperature referred herein is measured in accordance with JIS K-2269.

When the polyamine (1) is used in an asphalt emulsion, the polyamine (1) is used in the state of an aqueous solution of a water-soluble salt prepared from the polyamine (1) and an inorganic or organic acid.

Examples of the inorganic or organic acid to be used for the preparation of the water-soluble salt include hydrochloric acid, phosphoric acid, sulfuric acid, nitric acid, acetic acid and glycolic acid. Among them, hydrochloric acid and phosphoric acid are preferred. A preferable pH value of the aqueous solution of a water-soluble salt of the amine varies depending on the kinds of acid to be used. The pH value is preferably 7.0 or below from the standpoint of emulsifiability and emulsion stability. On the other hand, the pH value is preferably 1.0 or above from the standpoint of corrosion prevention of emulsifying machines, storage containers and the like, and also from the standpoint of costs associated with the use of a large amount of acids. More specifically, the pH value is preferably in the range of from 1.5 to 3.5 in the case where hydrochloric acid is used, the pH value is preferably in the range of from 1.5 to 4.0 in case where phosphoric acid is used, and the pH value is preferably in the range of from 4.0 to 7.0 in the case where acetic acid is used.

From the standpoint of emulsifiability and emulsion stability, the content of the water soluble salt of the polyamine (1) in the asphalt emulsion composition of the present invention is preferably 0.05 to 5.0%, more preferably 0.1 to 3.0% and most preferably 0.2 to 2.0% by weight based on the total weight of the asphalt emulsion composition. Besides, a preferable pH value of the asphalt emulsion is 1 to 7.

The asphalt emulsion composition of the present invention can be prepared by passing a water-soluble salt of the polyamine (1) and asphalt at the same time through an emulsifying machine such as a colloid mill. When the asphalt emulsion composition is prepared, the temperature of the asphalt is preferably 110 to 170°C and the temperature of the water-soluble salt of the amine is preferably 30 to 60°C.

The asphalt for use can be one ordinarily used for the paving of a road. Examples of the asphalt include straight asphalt, semi-blown asphalt, blown asphalt, polymer-modified asphalt, tar, coal tar and the like.

From the standpoint of better stability of the emulsion composition, the content of the asphalt in the asphalt emulsion composition is preferably 40% by weight or more based on the total weight of the asphalt emulsion composition. On the other hand, from the standpoint of better workability due to the viscosity of the emulsion composition which is not excessively high, the content of the asphalt in the asphalt emulsion composition is preferably 75% or less, more preferably 50 to 70% and most preferably 55 to 65% by weight based on the total weight of the asphalt emulsion composition.

In order to impart a high-level of durability to a road, the asphalt emulsion composition of the present invention preferably contains a polymer or latex for modification of asphalt.

Examples of the polymer for modification of asphalt include synthetic rubbers such as a styrene-butadiene rubber, a styrene-butadiene-styrene rubber, a chloroprene rubber and the like; thermoplastic resins such as an ethylene-vinyl acetate copolymer, an ethylene-ethyl acrylate copolymer and the like; and natural rubbers. Examples of the latex include a styrene-butadiene latex, a chloroprene latex, a neoprene latex and the like. The contents thereof are preferably 1 to 20 %, and more preferably 3 to 10 % by weight in the composition.

The methods, whereby a polymer or latex for modification of asphalt is incorporated into the asphalt composition of the present invention, include a method wherein asphalt modified with the polymer is used in the preparation of the emulsion composition; and a method wherein the latex is added into the water-soluble salt of the amine for use as an emulsifier, or wherein the latex is added into the emulsion composition. The latex may be added in so far as the addition of the latex does not impair the stability, emulsion stability, miscibility of aggregates at the time of paving operation, and the setting property after paving operation of the asphalt emulsion. Since the use of the latex modifies the asphalt remaining after paving operation as a result of the evaporation of water from the asphalt emulsion composition, the durability of road is remarkably improved.

Further, in order to improve storage stability and workability of the asphalt emulsion composition, additives or emulsification aids may be added to the asphalt emulsion composition. Examples of the additives or emulsification aids include alkylamines and alkylpolyamines; aliphatic amidoamines; alkylimidazolines; quaternary salts; nonionic surfactants such as a polyoxyalkylenealkylphenol; amphoteric surfactants such as alkylbetaine; higher fatty acids; higher alcohols; and inorganic salts such as calcium chloride, sodium chloride and potassium chloride. Further, the asphalt emulsion may contain a water-soluble polymer such as carboxymethyl cellulose, hydroxyethyl cellulose, polyvinyl alcohol or the like in order to improve storage stability and viscosity. Still further, the asphalt emulsion may contain a polyphenol compound such as tannin or the like in order to improve the adhesion between the aggregates and the asphalt. These additives and emulsification aids may be added to the asphalt emulsion in so far as the addition does not impair the stability, emulsion stability, miscibility of aggregates at the time of paving operation, and the setting property after paving operation of the asphalt emulsion.

Since the asphalt emulsion composition prepared as described above is excellent in miscibility with aggregates and in quick setting property, the asphalt emulsion composition as a quick setting asphalt is suited for use in a method such as slurry seal or micro-surfacing and for use in the repair of the sinking or cracking of road surface. The above-mentioned method comprises a step of mixing aggregates; water; a filler such as cement, slaked lime or the like; and an additive, in a vehicle, and another step of spreading the mixture on a road.

### Industrial Applicability

The change in content of the salt of the polyamine (1) in the asphalt emulsion composition of the present invention makes it possible to adjust physical properties such as miscibility of aggregates and setting property. Therefore, the asphalt emulsion composition is compatible with a wide range of aggregates. As is experienced in actual paving operation, even if the same aggregates are used, changes in external conditions such as temperature and in the particle size distribution of aggregates cause the time required for the mixing of asphalt emulsion composition with aggregates or the setting property after paving operation to vary. However, according to the present invention, time required for the mixing of asphalt emulsion composition with aggregates or setting property after paving operation can be easily adjusted to the requirements by slightly adjusting the amount of water to be added, the amount of cement or the like. As a result, the slurry seal or micro-surfacing operation can be drastically facilitated because the slurry seal or micro-surfacing operation can be exempted from prior minute adjustment of formulation and skill hitherto required.

### Brief Description of Drawings

Fig. 1 is a ¹³C-NMR spectrum of the stearyltetramine obtained in following Synthesis example 1.
Fig. 2 is a ¹³C-NMR spectrum of the tallow alkyltetramine obtained in following Synthesis example 2.

### Examples

In the synthesis examples, the solidification temperature was measured in accordance with JIS K-2269, and the viscosity was measured by using Broo k field viscometer (manufactured by TOKIMEC INC.). All parts are given by weight.

### Synthesis example 1

Stearylamine (270 g, 1 mole) was placed in a flask and the contents were heated to 60°C. Then, acrylonitrile (132.5 g, 2.5 moles) was added dropwise into the flask over 2 hours. After the completion of the addition, the reaction mixture was heated to 95°C and was stirred for 5 hours at that temperature. In this way, a cyanoethylation product was obtained. 350 g of the cyanoethylation product thus obtained and 4 g of Raney nickel were placed in an autoclave. The contents were heated to 130°C and hydrogen was introduced into the autoclave. In this way, a hydrogenation reaction was carried out for 5 hours by maintaining the pressure at a constant value of 1.47 MPa. Upon completion of the hydrogenation reaction, the reaction product was cooled, and the Raney nickel was removed by filtration. After purification by distillation, N-stearyl-N-aminopropylpropylenediamine (a compound represented by the formula (2) wherein R is a stearyl group) was obtained.

The amine (192 g, 0.5 mole) obtained in the above was placed in a flask and the contents were heated to 60°C. Then, acrylonitrile (13.3 g, 0.25 moles) was added dropwise into the flask over 2 hours. After the completion of the addition, the reaction mixture was heated to 95°C and was stirred for 5 hours at that temperature. In this way, a cyanoethylation product was obtained. 150 g of the cyanoethylation product thus obtained and 2 g of Raney nickel were placed in an autoclave. The contents were heated to 130°C and hydrogen was introduced into the autoclave. In this way, a hydrogenation reaction was carried out for 5 hours by maintaining the pressure at a constant value of 1.47 MPa. Upon completion of the hydrogenation reaction, the reaction product was cooled, and the Raney nickel was removed by filtration. After purification by distillation, stearyltetramine (a compound represented by the formula (1) wherein R is a stearyl group, x = y = 1 and z = 0) was obtained.

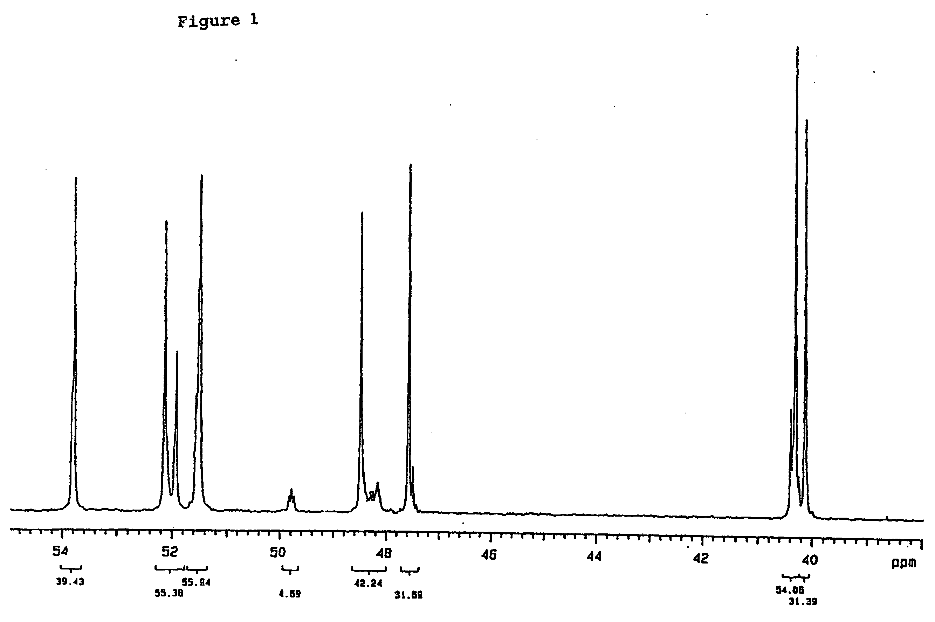

¹³C-NMR spectrum of the stearyltetramine obtained is shown in Fig. 1. The solidification temperature of the stearyltetramine was 20°C.

### Synthesis example 2

The procedure of Synthesis example 1 was repeated, except that tallow alkylamine (275 g, 1 mole) was used in place of the steraylamine. In this way, N-tallow alkyl-N-aminopropylpropylenediamine (a compound represented by the formula (2) wherein R is a tallow alkyl group) and, in a similar way that followed, tallow alkyltetramine (a compound represented by the formula (1) wherein R is a tallow alkyl group, x = y = 1 and z = 0) were obtained.

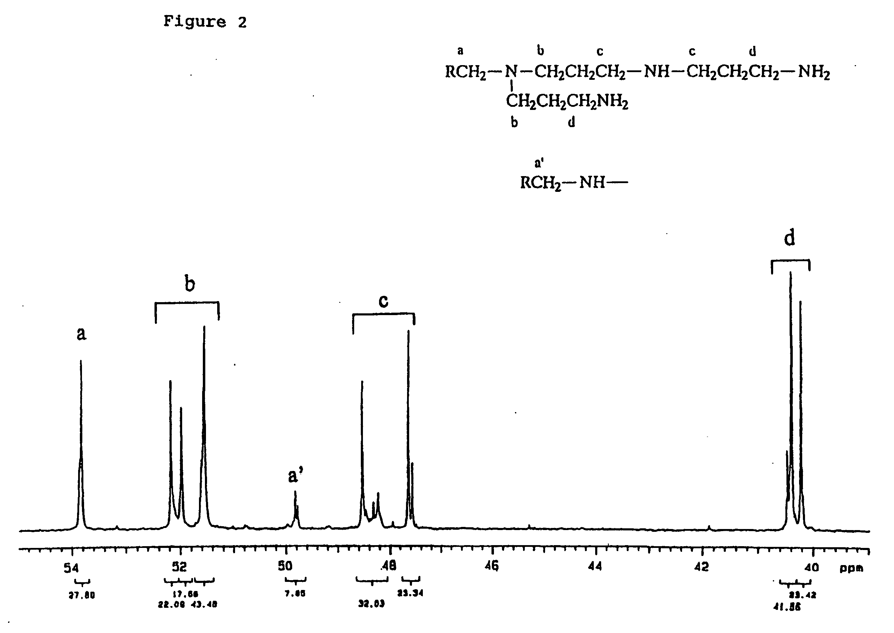

¹³C-NMR spectrum of the tallow alkyltetramine obtained is shown in Fig. 2. The solidification temperature of the tallow alkyltetramine was 3°C.

### Synthesis example 3

Stearylpropylenediamine (326 g, 1 mole) was placed in a flask and the contents were heated to 60°C. Then, acrylonitrile (169.6 g, 3.2 moles) was added dropwise into the flask over 2 hours. After the completion of the addition, the reaction mixture was heated to 95°C and was stirred for 5 hours at that temperature. In this way, a cyanoethylation product was obtained. 350 g of the cyanoethylation product thus obtained and 4 g of Raney nickel were placed in an autoclave. Further the following steps of Synthesis example 3 were conducted as same as Synthesis example 1, and stearylpentamine (a compound represented by the formula (1) wherein R is a stearyl group and x = y = z =1) was obtained.

The solidification temperature of the stearylpentamine was 17°C.

### Synthesis example 4

Tallow alkylpropylenediamine (332 g, 1 mole) was placed in a flask and the contents were heated to 60°C. Then, acrylonitrile (111.3 g, 2.1 moles) was added dropwise into the flask over 2 hours. After the completion of the addition, the reaction mixture was heated to 95°C and was stirred for 5 hours at that temperature. In this way, a cyanoethylation product was obtained.

Next, 350 g of the cyanoethylation product thus obtained and 4 g of Raney nickel were placed in an autoclave. The hydrogenation reaction of the contents were conducted in the same condition as Synthesis example 1. Upon completion of the hydrogenation reaction, the reaction product was cooled, and the Raney nickel was removed by filtration. Thus, a desired liquid amine (hereinafter referred to as product 1 of the present invention) was obtained.

The liquid amine contained as a main component 54% by weight of a compound represented by the formula (1) wherein R is a tallow alkyl group, x = y = 1 and z = 0. The amine was a liquid having a viscosity of 150 mPa•s at 25°C and having a solidification temperature of 14°C.

### Synthesis example 5

The procedure of Synthesis example 4 was repeated, except that tallow alkylpropylenediamine (332 g, 1 mole) and acrylonitrile (132.5 g, 2.5 moles) were used. Thus, a desired liquid amine (hereinafter referred to as product 2 of the present invention) was obtained.

The liquid amine contained as a main component 57% by weight of a compound represented by the formula (1) wherein R is a tallow alkyl group, x = y = 1 and z - 0. The amine was a liquid having a viscosity of 120 mPa•s at 25°C and having a solidification temperature of 10°C.

### Synthesis example 6

The procedure of Synthesis example 4 was repeated, except that tallow alkylpropylenediamine (332 g, 1 mole) and acrylonitrile (84.8 g, 1.6 moles) were used. Thus, a desired liquid amine (hereinafter referred to as product 3 of the present invention) was obtained.

The liquid amine contained as a main component 34% by weight of a compound represented by the formula (1) wherein R is a tallow alkyl group, x = y = 1 and z = 0. The amine was a liquid having a viscosity of 180 mPa•s at 25°C and having a solidification temperature of 18°C.

### Synthesis example 7

The procedure of Synthesis example 4 was repeated, except that stearylpropylenediamine (326 g, 1 mole) and acrylonitrile (138.7 g, 2.6 moles) were used. Thus, a desired liquid amine (hereinafter referred to as product 4 of the present invention) was obtained.

The liquid amine contained as a main component 58% by weight of a compound represented by the formula (1) wherein R is a stearyl group, x = y = 1 and z = 0. The amine was a liquid having a viscosity of 95 mPa•s at 25°C and having a solidification temperature of 9°C.

### Synthesis example 8

The procedure of Synthesis example 4 was repeated, except that tallow alkyldipropylenetriamine (389 g, 1 mole) and acrylonitrile (169.6 g, 3.2 moles) were used. Thus, a desired liquid amine (hereinafter referred to as product 5 of the present invention) was obtained.

The liquid amine contained as a main component 44% by weight of a compound represented by the formula (1) wherein R is a tallow alkyl group, x = 1, y = 2 and z = 1. The amine was a liquid having a viscosity of 78 mPa•s at 25°C and having a solidification temperature of 5°C.

### Synthesis example 9

The procedure of Synthesis example 4 was repeated, except that N,N-di(aminopropyl)tallow alkylamine (389 g, 1 mole) and acrylonitrile (53.0 g, 1 mole) were used. Thus, a desired liquid amine (hereinafter referred to as product 6 of the present invention) was obtained.

The liquid amine contained as a main component 48% by weight of a compound represented by the formula (1) wherein R is a tallow alkyl group, x = y = 1 and z = 0. The amine was a liquid having a viscosity of 140 mPa•s at 25°C and having a solidification temperature of 8°C.

### Comparative synthesis example 1

As in Synthesis example 4, tallow alkylpropylenediamine (332 g, 1 mole) and acrylonitrile (53.0 g, 1.0 mole) were used, and cyanoethylation and hydrogenation reactions were carried out to obtain a reaction product. After being cooled to 60°C, a cyanoethylation of the reaction product was carried out by replacing the hydrogen in the system with nitrogen and by using acrylonitrile (53.0 g, 1.0 mole). Consecutively, a hydrogenation reaction was carried out. Further, a cyanoethylation reaction was carried out by using acrylonitrile (26.5 g, 0.5 mole), and consecutively a hydrogenation reaction was carried out. Next, Raney nickel was removed by filtration from the reaction product. In this way, a comparative product 1 was obtained.

The comparative product 1 was a mixture of compounds represented by the formula (1) wherein R is a tallow alkyl group, x = z = 0, y = 2 and/or y = 3, and was a solid at 25°C having a solidification temperature of 45°C.

It can be seen from the above description that the solidification temperatures of the products of the present invention are far below than the solidification temperature of the comparative product 1 and that the products of the present invention are superior in handling property because these products are in a liquid state at 20°C.

### Example 1

Water was added to 0.6 parts of the product 1 of the present invention and the pH value was adjusted to 2. 0 by using hydrochloric acid. In this way, 38 parts of an aqueous solution of a hydrochloric acid salt of the amine was prepared. The total amount of the solution was heated to 40°C. Then, the solution and 62 parts of asphalt having a penetration of 60 to 80 and fused at 145°C were passed at the same time through a colloid mill to thereby prepare an emulsion composition A. Evaporation residue, viscosity, storage stability and residue on sieve were measured by respective methods in accordance with ASTM D-3910. The results are shown in Table 1.

### Examples 2 to 12

Emulsion compositions B to L were prepared by repeating the procedure of Example 1, except that the emulsifier and the amount thereof were changed as shown in Table 1 to evaluate the performances. The results are shown in Table 1.

### Comparative Examples 1 and 2

Emulsion compositions a and b were prepared by repeating the procedure of Example 1, except that a comparative product 1 in amounts as shown in Table 1 was used in place of the product 1 of the present invention. The performances were evaluated. The results are shown in Table 1.

### Comparative Examples 3 to 8

Emulsion compositions c to h were prepared by repeating the procedure of Example 1, except that the amines were those generally used as emulsifiers for quick setting asphalt, i.e., a product obtained by quaternizing tallow alkyl propylenediamine with methyl chloride (a comparative product 2), a tall oil fatty acid amidoamine obtained from tall oil fatty acid and pentaethylenehexamine (a comparative product 3) and N,N-di(aminopropyl)tallow alkylamine (a comparative product 4) in respective amounts as shown in Table 1 in place of the product 1 of the present invention. The performances were evaluated. The results are shown in Table 1.

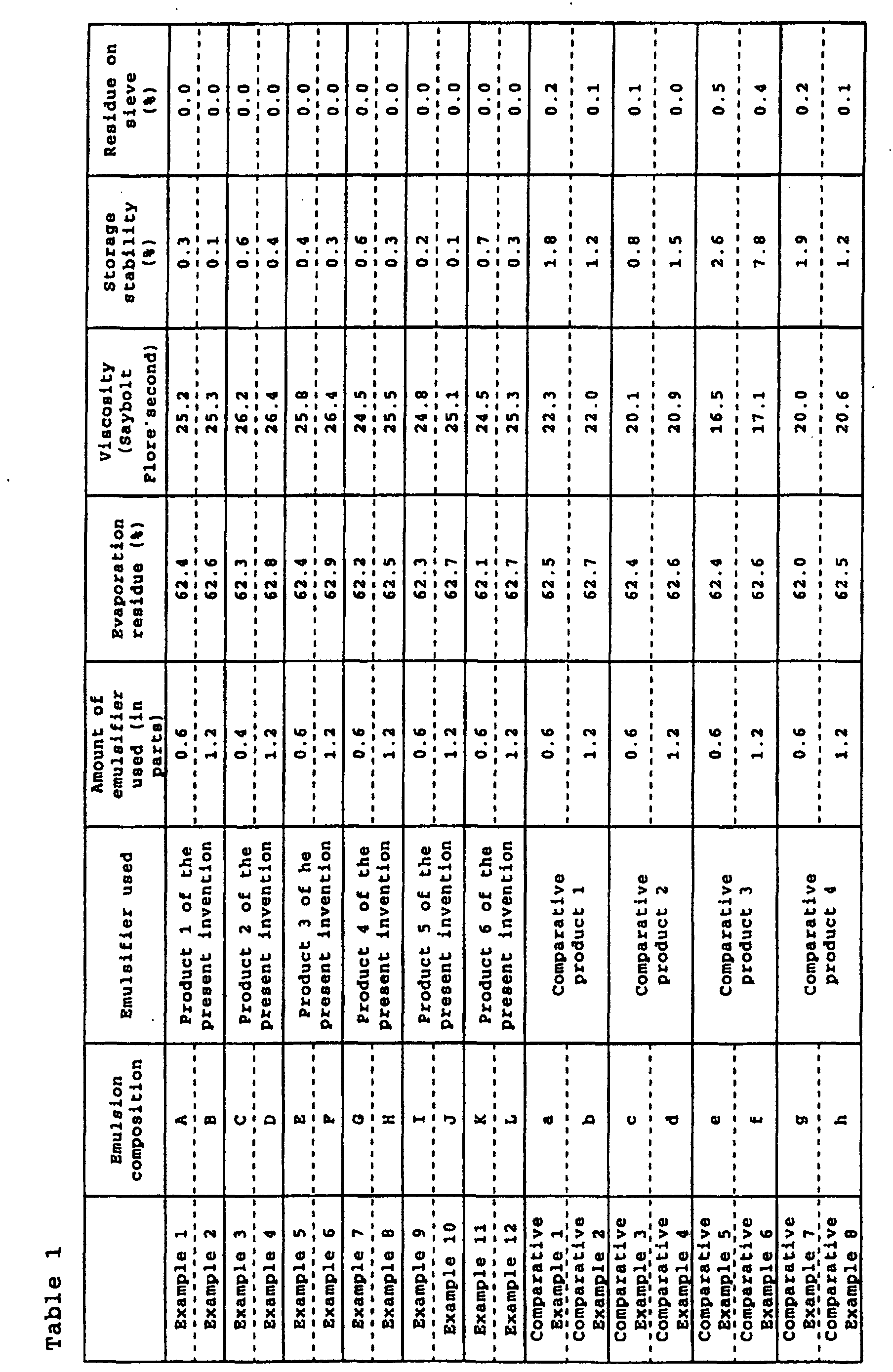

As is apparent from Table 1, in contrast with the emulsion compositions of Comparative Examples, the emulsion compositions of the present invention are superior in storage stability and produce no residue on sieve and thus the emulsion compositions of the present invention are satisfactory as asphalt emulsion compositions.

### Examples 13 to 24, and Comparative Examples 9 to 16

By using the emulsion compositions A to L, and emulsion compositions a to h obtained in Examples 1 to 12 and Comparative Examples 1 to 8, properties as quick setting slurry seal were measured and evaluated in accordance with the methods described in Design Technical Bulletins issued from International Slurry Surfacing Association (ISSA). The aggregates used for the evaluation were granite and hard sandstone produced in Mexico. The particle size distributions complied with Type II of ISSA A105. The filler used was Portland cement. Mixing time of aggregates was evaluated in accordance with the method described in ISSA No. 102. A longer mixing time is desirable, because a longer mixing time means a better workability and ensures a work life. In order to ensure sufficient mixing between the emulsion and the aggregates and to spread and flatten the mixture, the mixing time needs to be 2 minutes or longer. In addition, in accordance with the method described in ISSA No. 139, the strengths of the mixture sampled 30 minutes later and 60 minutes later were measured to thereby evaluate the quick setting property. The sample whose strength exceeds 12 kg-cm after 30 minutes can be rated as quick setting, and the sample whose strength exceeds 20 kg-cm after 60 minutes can be rated as quick-traffic. A higher strength is desirable, because a paving material having a higher strength can be open to traffic in a shorter period of time. The requirements for both mixing time and quick setting property need to be fulfilled.

Evaluation results are shown in Table 2.

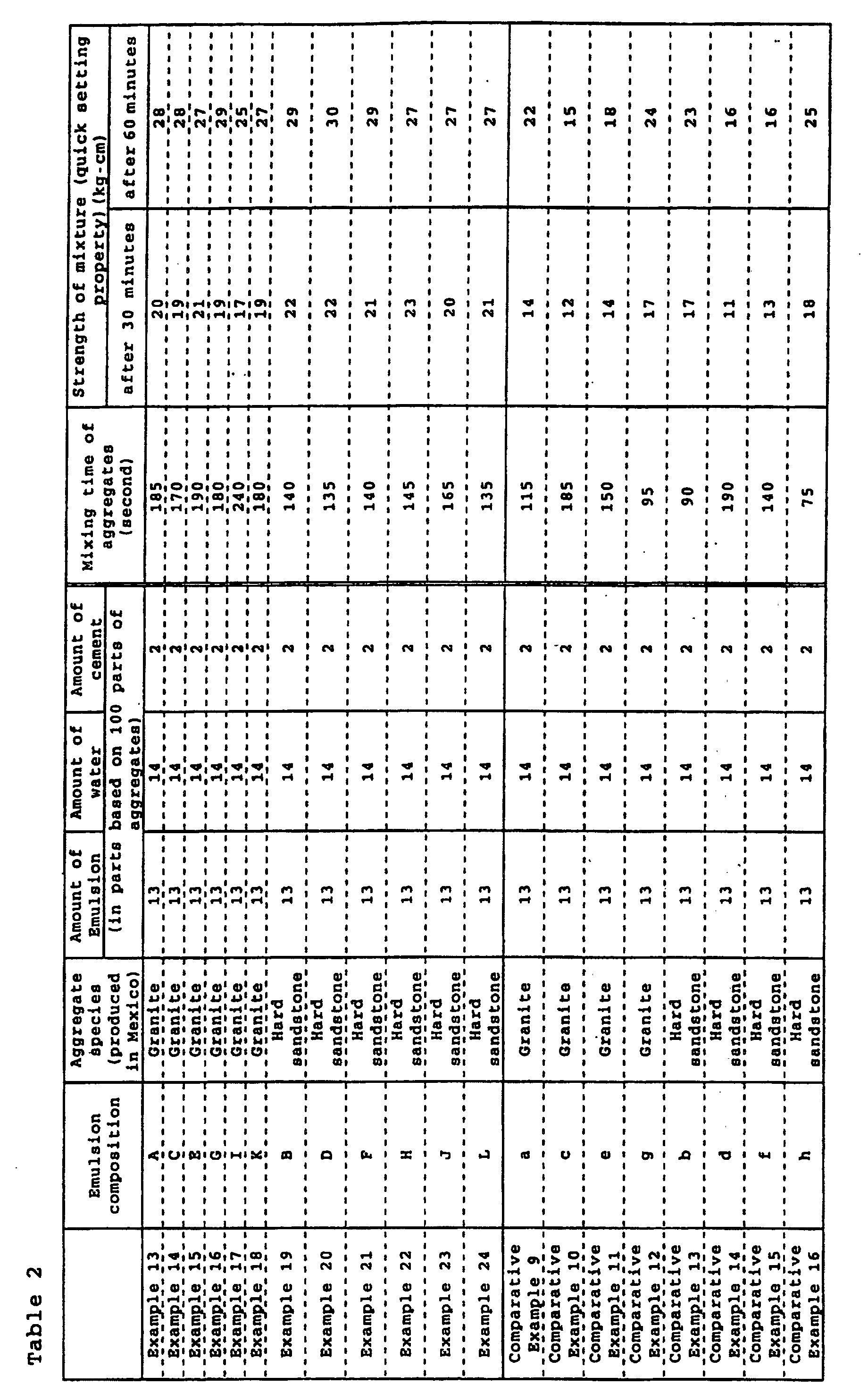

It can be seen from the results of Table 2 that the emulsion compositions of the present invention exhibit good miscibility with aggregates and quick setting property for both granite and hard sandstone. Therefore, the emulsion compositions of the present invention can be open to traffic in a shorter period of time in contrast with the emulsion compositions of Comparative Examples.

### Examples 25 to 30, and Comparative Examples 17 to 22

The procedures of Examples 15 and 23 and Comparative Examples 11 and 14 were repeated, except that the amounts of cement and water to be added to the mixtures were changed as shown in Table 3. The results are shown in Table 3.

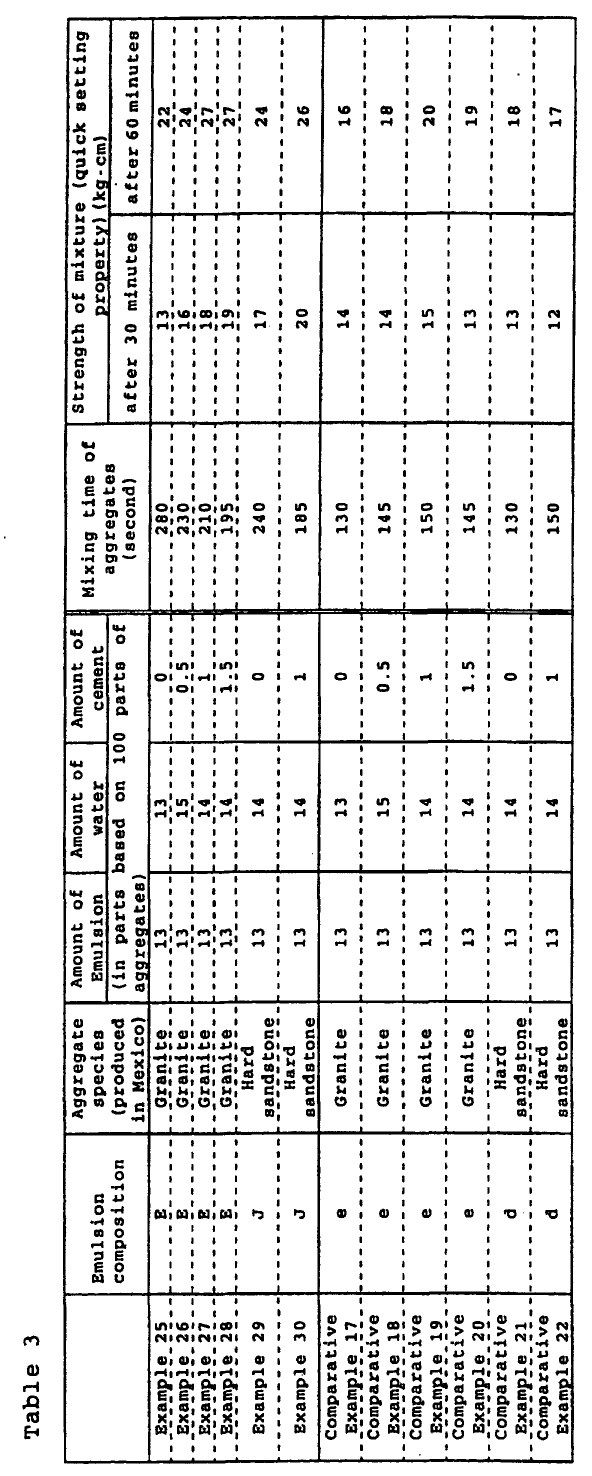

It can be seen from the results of Table 3 that the emulsion compositions of the present invention exhibit good miscibility and quick setting property even if the amounts of cement change. Therefore, it is possible to adjust the mixing time by the amount of cement while securing sufficient quick setting property.

## Claims

1. A salt of a polyamine, preparable from a polyamine represented by the formula (1) and an inorganic or organic acid: 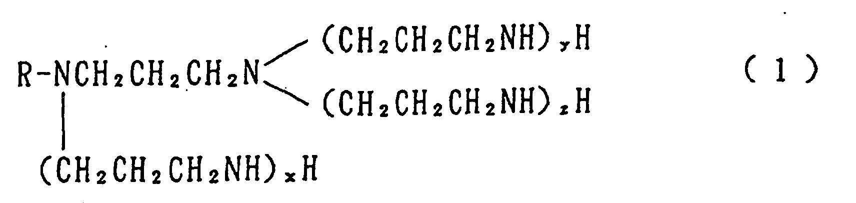 wherein R is a straight or branched hydrocarbon group having 8 to 22 carbon atoms; x is a number of 1 to 5; and each of y and z is a number of 0 to 5 with the proviso that both of y and z are not 0 at the same time.

2. A process for producing the polyamine salt as claimed in Claim 1, comprising the steps of carrying out the cyanoethylation of a compound represented by the formula (2) or (3) by reacting acrylonitrile with the compound and hydrogenating the cyanoethylation product, and reacting the hydrogenated product with an inorganic or organic acid: 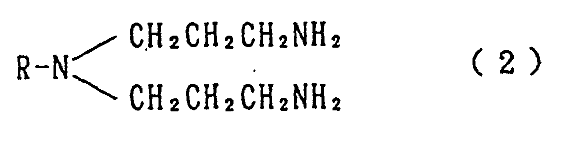
R-(NHCH₂CH₂CH₂)ₘ-NH₂ (3)
wherein R is as defined above; and m is a number of 1 to 3.

3. A salt of a polyamine for use in an asphalt emulsion as claimed in claim 1, said amine being obtainable by carrying out the cyanoethylation of a compound represented by the formula (2) by reacting 0.2 to 3 moles of acrylonitrile with 1 mole of the compound or carrying out the cyanoethylation of a compound represented by the formula (3) by reacting 1.4 to (m+2) moles of acrylonitrile with 1 mole of the compound; and hydrogenating the cyanoethylation product.

4. An asphalt emulsion composition containing a salt of the polyamine as claimed in Claim 1 or 3.

5. The composition as claimed in Claim 4, wherein the content of the salt of the polyamine is 0.05 to 5.0% by weight based on the total weight of the asphalt emulsion composition.

6. A method of emulsifying asphalt with a salt of the polyamine as defined in Claim 1 or 3.

7. A method of emulsifying asphalt with the polyamine as defined Claim 1 or 3 and an acid.

8. Use of a salt of the polyamine as defined Claim 1 or 3 as an emulsifier for asphalt.

9. An asphalt emulsion comprising asphalt, water and a salt of the polyamine as defined in Claim 1 or 3.

## Patentansprüche

1. Polyaminsalz, herstellbar aus einem durch die Formel (1) dargestellten Polyamin und einer anorganischen oder organischen Säure: 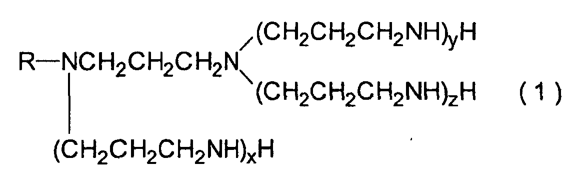 wobei R eine geradkettige oder verzweigte Kohlenwasserstoffgruppe mit 8 bis 22 Kohlenstoffatomen ist; x eine Zahl von 1 bis 5 ist; und sowohl y als auch z eine Zahl von 0 bis 5 sind, unter der Massgabe, dass y und z nicht gleichzeitig 0 sind.

2. Verfahren zur Herstellung des Polyaminsalzes gemäss Anspruch 1, umfassend die Schritte der Durchführung der Cyanoethylierung einer durch die Formel (2) oder (3) dargestellten Verbindung durch Umsetzung von Acrylnitril mit der Verbindung und Hydrieren des Cyanoethylierungsprodukts und Umsetzen des hydrierten Produkts mit einer anorganischen oder organischen Säure: 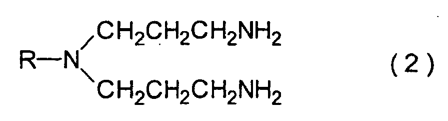
R―(NHCH₂CH₂CH₂)ₘ―NH₂ (3)
wobei R wie oben definiert ist; und m eine Zahl von 1 bis 3 ist.

3. Polyaminsalz zur Verwendung in einer Asphaltemulsion gemäss Anspruch 1, wobei das Amin erhältlich ist durch Durchführung der Cyanoethylierung einer durch die Formel (2) dargestellten Verbindung durch Umsetzen von 0,2 bis 3 mol Acrylnitril mit 1 mol der Verbindung oder durch Durchführung der Cyanoethylierung einer durch die Formel (3) dargestellten Verbindung durch Umsetzen von 1,4 bis (m+2) mol Acrylnitril mit 1 mol der Verbindung; und Hydrieren des Cyanoethylierungsprodukts.

4. Asphaltemulsionszusammensetzung, enthaltend ein Polyaminsalz gemäss Anspruch 1 oder 3.

5. Zusammensetzung gemäss Anspruch 4, wobei der Gehalt an Polyaminsalz 0,05 bis 5,0 Gew.%, in bezug auf das Gesamtgewicht der Asphaltemulsionszusammensetzung, beträgt.

6. Verfahren zum Emulgieren von Asphalt mit einem Polyaminsalz, das gemäss Anspruch 1 oder 3 definiert ist.

7. Verfahren zum Emulgieren von Asphalt mit dem Polyamin, das gemäss Anspruch 1 oder 3 definiert ist, und einer Säure.

8. Verwendung eines Polyaminsalzes, das gemäss Anspruch 1 oder 3 definiert ist, als Emulgator für Asphalt.

9. Asphaltemulsion, umfassend Asphalt, Wasser und ein Polyaminsalz, das gemäss Anspruch 1 oder 3 definiert ist.

## Revendications

1. Sel d'une polyamine, pouvant être préparé à partir d'un polyamine représenté par la formule (1) et d'un acide inorganique ou organique : 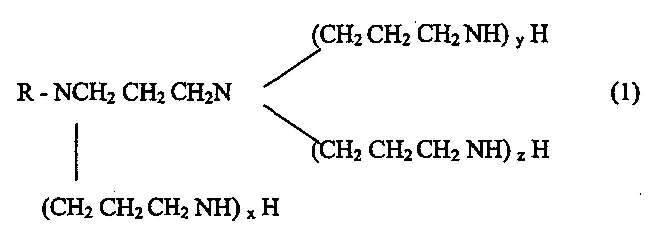 où R est un groupe hydrocarbure droit ou ramifié possédant 8 à 22 atomes de carbone ; x est un nombre de 1 à 5 ; et chacun de y et z est un nombre de 0 à 5 à condition que y et z ne soient pas égaux à 0 en même temps.

2. Procédé pour produire le sel de polyamine selon la revendication 1, comprenant les étapes de réalisation de la cyanoéthylation d'un composé représenté par la formule (2) ou (3) en faisant réagir de l'acrylonitrile avec le composé et en hydrogénant le produit de la cyanoéthylation, et en faisant réagir le produit hydrogéné avec un acide inorganique ou organique : 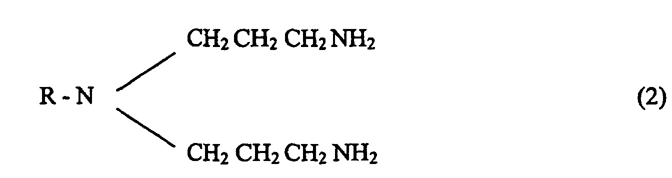
R - (NHCH₂ CH₂ CH₂)ₘ-NH₂ (3)
où R est tel que défini ci-dessus ; et m est un nombre de 1 à 3.

3. Sel d'une polyamine pour utilisation dans une émulsion d'asphalte selon la revendication 1, ladite amine pouvant être obtenue en effectuant la cyanoéthylation d'un composé représenté par la formule (2) en faisant réagir 0,2 à 3 moles d'acrylonitrile avec 1 mole du composé ou en effectuant la cyanoéthylation d'un composé représenté par la formule (3) en faisant réagir 1,4 à (m + 2) moles d'acrylonitrile avec 1 mole du composé ; et en hydrogénant le produit de la cyanoéthylation.

4. Composition d'émulsion d'asphalte contenant un sel de la polyamine selon la revendication 1 ou 3.

5. Composition selon la revendication 4, dans laquelle la teneur en sel de la polyamine est 0,05 à 5% en poids basée sur le poids total de la composition d'émulsion d'asphalte.

6. Procédé de mise en émulsion d'asphalte avec un sel de la polyamine selon la revendication 1 ou 3.

7. Procédé de mise en émulsion d'asphalte avec la polyamine selon la revendication 1 ou 3 et un acide.

8. Utilisation d'un sel de la polyamine selon la revendication 1 ou 3 comme émulsifiant pour l'asphalte.

9. Emulsion d'asphalte comprenant de l'asphalte, de l'eau et un sel de la polyamine selon la revendication 1 ou 3.
